(19) Europäisches Patentamt
European Patent Office
Office européen des brevets

(11) Publication number: **0 212 213 B1**

# EUROPEAN PATENT SPECIFICATION

(45) Date of publication of patent specification: 29.05.91

(51) Int. Cl.⁵: **A61M 25/02, A61B 19/00, A61M 5/00, A61M 1/00**

(21) Application number: **86109456.3**

(22) Date of filing: **10.07.86**

(54) **Cerebral catheterization apparatus.**

(30) Priority: **16.07.85 US 755427**

(43) Date of publication of application:
**04.03.87 Bulletin 87/10**

(45) Publication of the grant of the patent:
**29.05.91 Bulletin 91/22**

(84) Designated Contracting States:
**AT BE CH DE FR GB IT LI LU NL SE**

(56) References cited:
**DE-A- 2 911 912**     **US-A- 2 616 429**
**US-A- 2 670 730**     **US-A- 3 457 922**
**US-A- 3 938 530**     **US-A- 4 315 513**
**US-A- 4 445 886**     **US-A- 4 500 311**
**US-A- 4 516 968**

(73) Proprietor: **THOMAS JEFFERSON UNIVERSITY**
**11th and Walnut Streets**
**Philadelphia Pennsylvania 19107(US)**

(72) Inventor: **Osterholm, Jewell L.**
**549 Huston Road**
**Radnor Pennsylvania(US)**

(74) Representative: **Baillie, Iain Cameron et al**
**c/o Ladas & Parry Isartorplatz 5**
**W-8000 München 2(DE)**

## Description

This invention relates in general to cerebral and lumbar catheterization apparatus useful for the perfusion or drainage of fluids from the cerebral and spinal regions and, in particular, it relates to an apparatus particularly useful in treating hypoxic/ischemic neurologic tissue arising from cerebral vascular accident by allowing the circulation of oxygen carrying synthetic liquids through the neurologic tissue.

This application relates to U.S. Patent 4,445,500, U.S. Patent 4,445,886, U.S. Patent 4,378,797 and also to U.S. Patent 4,445,514 entitled "Extra Vascular Circulation of Oxygenated Synthetic Nutrients to Treat Tissue Hypoxic and Ischemic Disorders", U.S. Patent 4,393,863, entitled "Extra Vascular Circulation of Oxygenated Synthetic Nutrients to Treat Tissue Hypoxic and Ischemic Disorders", U.S. Patent 4,450,841, entitled "Stroke Treatment Utilizing Extra Vascular Circulation of Oxygenated Synthetic Nutrients to Treat Tissue Hypoxic and Ischemic Disorders", U.S. Patent 4,445,887, entitled "Stroke Treatment Utilizing Extra Vascular Circulation of Oxygenated Synthetic Nutrients to Treat Tissue Hypoxic and Ischemic Disorders", U.S. Patent 4,446,154, entitled "Stroke Treatment Utilizing Extra Vascular Circulation of Oxygenated Synthetic Nutrients to Treat Tissue Hypoxic and Ischemic Disorders", U.S. Patent 4,446,155, entitled "Stroke Treatment Utilizing Extra Vascular Circulation of Oxygenated Synthetic Nutrients to Treat Tissue Hypoxic and Ischemic Disorders", U.S. Patent 4,451,251, entitled "Stroke Treatment Utilizing Extra Vascular Circulation of Oxygenated Synthetic Nutrients to Treat Tissue Hypoxic and Ischemic Disorders", U.S. Patent 4,445, 888, entitled "Stroke Treatment Utilizing Extra Vascular Circulation of Oxygenated Synthetic Nutrients to Treat Tissue Hypoxic and Ischemic Disorders", U.S. Patent 4,445,500, entitled "Stroke Treatment Utilizing Extra Vascular Circulation of Oxygenated Synthetic Nutrients to Treat Tissue Hypoxic and Ischemic Disorders".

U.S. Patent 4,500,311 and 3,457,922 are concerned with a catheter drainage assembly but there is neither provision nor suggestion of means to accurately locating and applying said catheter in a non-hospital environment.

Cerebral vascular accident, a disease commonly known as "stroke" remains the third leading cause of death, and probably constitutes the single largest category of long-term disability in the United States. In spite of current medical knowledge and available treatments, a major central system vascular occlusion is quickly attended by irreversible damage to the affected brain region(s). A "completed stroke" is manifest by a fixed and permanent neurological deficit. Millions of dollars have been expended in stroke research and care by federal and private agencies without a single substantial gain in our present chemotherapeutic abilities for a completed stroke.

In each of the aforementioned patents, however, the present applicant has disclosed and claimed a novel nutrient formulation for circulation through cerebro-spinal fluid pathways, and systems and methods for using the same to treat central nervous tissue hypoxic-ischemic conditions. Applicant as recognized that there is a therapeutic time window through which neurons can be reached and resuscitated.

In accordance with the inventions disclosed and claimed in the afore mentioned patents and applications, a synthetic cerebro-spinal fluid is circulated through the cerebro-spinal fluid passageways immediately after a stroke. Specifically, the oxygenated nutrient is circulated through those cerebro-spinal fluid passageways which contact brain and spinal cord tissue. According to these methods, treated tissues exhibit a substantially improved ability to resist and/or repair damage which would otherwise result from vascular occlusion.

The circulation apparatus disclosed in the aforementioned patents and applications comprises a reservoir containing a nutrient emulsion; means for delivering the nutrient emulsion at preselected flow rates; an oxygenation means for equilibrating the nutrient emulsion to desired gaseous tension levels; heat exchanger and/or cooling unit means for selectively controlling the temperature of the nutrient emulsion; filter means for cleansing the nutrient emulsion; and circulation monitoring means for ensuring the desired circulation flows are maintained within the system. Specifically, in carrying out the method described and claimed in the aforementioned patents, the nutrient emulsion is delivered from the nutrient emulsion reservoir to the lateral ventricle of the brain. Injection of the nutrient input stream directly to the brain permits the oxygenated nutrient emulsion to come into contact with the subarachnoid spaces, miniature Virchow-Robins spaces, cerebral and cord surfaces, and cerebral ventricles. Circulation of the nutrient input stream through at least a portion of the cerebro-spinal pathway may be accomplished by withdrawing fluid from the spinal subarachnoid space, or alternatively, from the cisterna magna.

While the aforementioned system and method have been found to be particularly useful in the treatment of stroke, treatment must be begun within a therapeutic time window immediately following the onset of the stroke. Because time is so critical, treatment must be administered by paramedics and emergency personnel or, alternatively, by emergency room physicians and staff. Since the treatment described in the aforementioned patents and applications requires the injection of oxygenated nutrient emulsion directly into the lateral ventricles of the human brain and the withdrawal of that nutrient emulsion from the subarchnoid space or the cisterna magna, it should be clear that the procedures used in the treatment are not ones which paramedics and emergency personnel or even most emergency room personnel are familiar with.

Specifically, the treatment requires that an aperture be formed in the cranium and that a catheter be inserted directly through the aperture of the skull and thereafter through the soft tissue of the brain into the lateral ventricles. Such techniques are difficult enough for skilled neurosurgeons and are typically beyond the ken of paramedical personnel.

According to the present invention there is provided a cerebral catheterization apparatus for rapidly inserting a cerebral perfusion catheter means into the lateral brain ventricle of a patient to permit rapid delivery of oxygenated nutrient to the cerebro-spinal fluid of the patient including an appliance for engaging the head, and guide means coupled to said appliance for receiving and directing said catheter means to the lateral brain ventricle, characterized in that the guide means is mounted on a harness positionable with respect to the head of the patient for positioning said guide means with respect to the head such that said catheter means may be reliably inserted into said lateral ventricle.

The appliance accurately positions the guide means with respect to the head at a location offset from the center line. The guide means is a tubular structure having a threaded internal bore. The catheter further includes an external threaded portion mating with the internal threaded bore of the guide means such that when the catheter hand guide means are mated, the insertion depth of the catheter may be precisely controlled by the rotational position of the catheter with respect to the guide means. A locking means is provided to ensure that once the catheter is inserted into the lateral ventricle, the position of the catheter vis-a-vis the guide means remains fixed during treatment so that the insertion depth does not vary during treatment.

The aforementioned placement appliance having guide means is applied to the head of the patient such that the guide means are positioned at a location offset from the center line of the head, with the center line of the bore of the aforementioned guide means being directed toward the lateral ventricles. An aperture is created through the skull at the location of the guide means and thereafter the catheter is inserted through the guide means and the skull using the guide means to direct the catheter toward the lateral brain ventricle.

The present invention will be more fully understood by reference to the accompanying drawings, in which:

Fig. 1 is a frontal view of a patient wearing a cerebral catheterization apparatus in accordance with the present invention;

Fig. 2 is a side or lateral view of the patient wearing the cerebral catheterization apparatus of Fig. 1, but with the catheter means removed therefrom;

Fig. 3 is a top view of the patient and cerebral catheterization apparatus shown in Fig. 2;

Fig. 4 is a partial cross-sectional view through the cerebral catheterization apparatus shown in Fig. 3 but with an obturator situated within the guide means thereof;

Fig. 5 is a cross-sectional view similar to that shown in Fig. 4, but with a drill means situated within the guide means thereof;

Fig. 6 is a cross-sectional view similar to that shown in Fig. 4, but with the catheter means of the present invention situated within the guide means, the catheter having a tip portion of a first configuration;

Fig. 7 is a schematic view of a lumbar catheterization apparatus useful in connection with the cerebral catheterization apparatus of the present invention;

Fig. 8 illustrates the tip of both the cerebral and the lumbar catheter means of the present invention in a first position suitable for insertion; and

Fig. 9 illustrates the tip of both the cerebral and the lumbar catheter means of the present invention but in a second position after insertion.

In the following more detailed description of the present invention, numerous examples have been selected for the purpose of explanation and illustration of the preferred embodiment. One of ordinary skill in the art will readily recognize that numerous substitutions or alterations from the embodiment set forth may be made without departing from the present invention, which is defined in the appended claims.

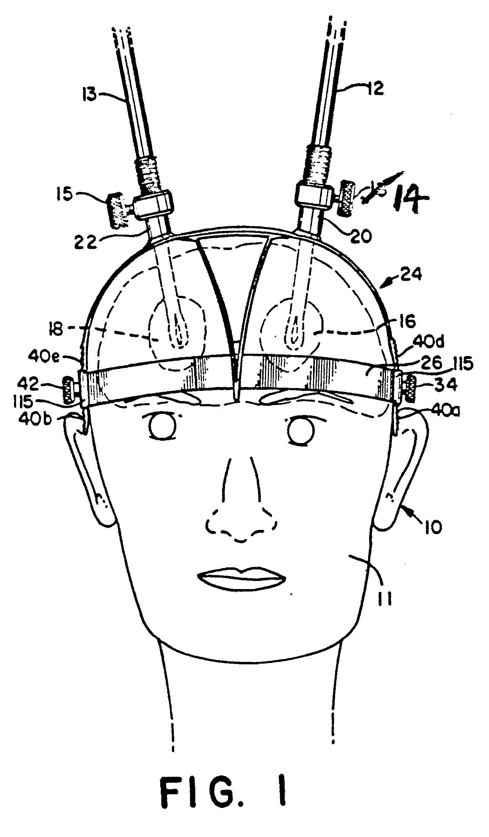

Referring now to Fig. 1, the cerebral catheterization apparatus of the present invention is shown generally at 10. The cerebral catheterization apparatus 10 is shown fitted on to a patient 11. The apparatus 10 is utilized for reliably inserting a cerebral perfusion catheter means 12 and 13 to the left and right lateral brain ventricles 16 and 18 of the patient 11. As shown in Fig. 1, the cerebral catheterization apparatus 10 includes an appliance for engaging the head having guide means shown generally at 20 and 22 for receiving and directing the catheter means 12 and 13.

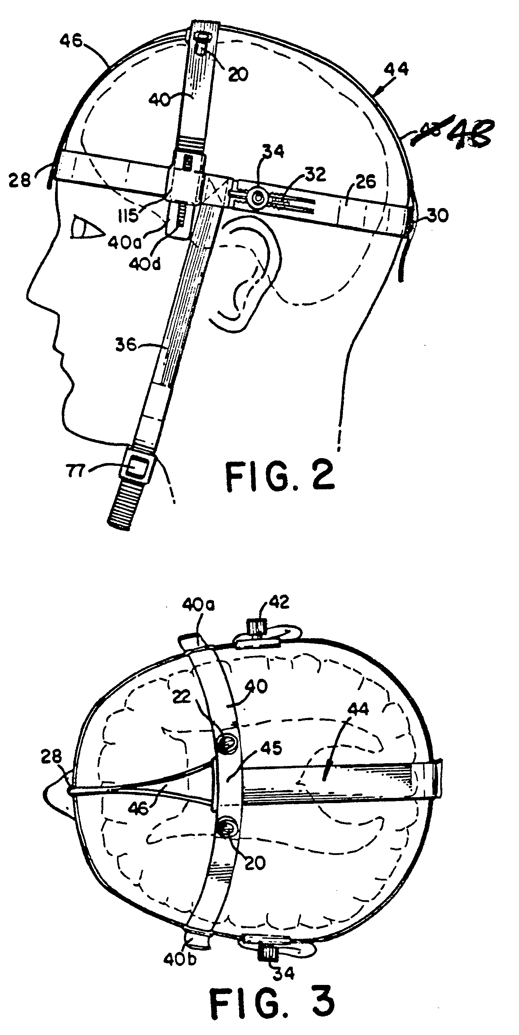

In order that the guide means 20 and 22 may be quickly and reliably positioned with respect to the left and right lateral brain ventricles 16 and 18 by emergency personnel, the guide means 20 and 22 are affixed to a harness 24 which accurately positions them with respect to the head of the patient 11. The harness 24 includes a horizontal strap 26 which is positioned about the head slightly above the ears and eyebrows. As best seen in Fig. 2, therefore, the horizontal strap 26 is positioned adjacent the forehead 28 at the anterior portion of the head and is positioned adjacent the occipital protuberance 30 at the posterior portion of the head. The horizontal strap 26 is thus positioned with respect to the head such that the vertex of the skull is located above the strap. The horizontal strap 26 includes a fastening means 32 which preferably includes a thumb screws 34 and 42 permitting the circumferential size of the horizontal strap 26 to be adjusted depending on the head size of the patient 11. When fitted on a patient, the horizontal strap is adjusted using both of these screws to take up the same abount of strap slack, thereby establishing (bilateral) symmetry of the head to the apparatus 10.

The harness 24 further includes chin straps, one of which is shown at 36, for maintaining the horizontal strap 26 in a fixed position with respect to the head. The chin straps are affixed to the horizontal strap 26 on the left and right sides of the head of the patient 11. The chin straps are joined by a buckle or other fastening means beneath the chin. The harness 24 further includes a vertical transverse strap 40 which is also affixed to the horizontal strap 26 and to which the guide means 20 and 22 are affixed. Like the horizontal strap 26, the vertical transverse strap 40 also includes adjustment means, such as racheted slides, to accommodate varying head sizes. This may be accomplished by providing indicia on each tongue 40a,40b of the strap 40 which indicate its position relative to its strap guide 115 which is attached to the horizontal strap 26. The tongues 40a and 40b may have ratchet racks 40d and 40e protruding from one surface thereof, to selectively engage complimentally formed protrusions or teeth on the strap guides 115. This engagement permits rapid adjustment of the strap 40, but holds it firmly in place once properly positioned. The vertical transverse strap 40 is generally positioned parallel to and lies directly above the coronal suture, i.e, the juncture between the frontal and parietal skull bones.

As may best be seen in Fig. 3, also joined to the horizontal strap 26 is a vertical medial strap shown generally at 44. The vertical medial strap 44 includes a first or anterior portion 46 which joins the horizontal strap 26 in the vicinity of the forehead at 28 and a second or posterior portion 48 which joins with the horizontal strap 26 in the vicinity of the occipital protuberance 30. The first or anterior portion 46 and the second or posterior portion 48 of the vertical medial strap 44 join with the vertical transverse strap 40 in a position 45 which is intermediate the guide means 20 and 22. Such a position lies roughly over the bregma, i.e., the juncture between the coronal and saggital sutures of the skull. The vertical medial strap 44 is effective in appropriately positioning the guide means 20 and 22 in the anterior-to-posterior direction over the bregma.

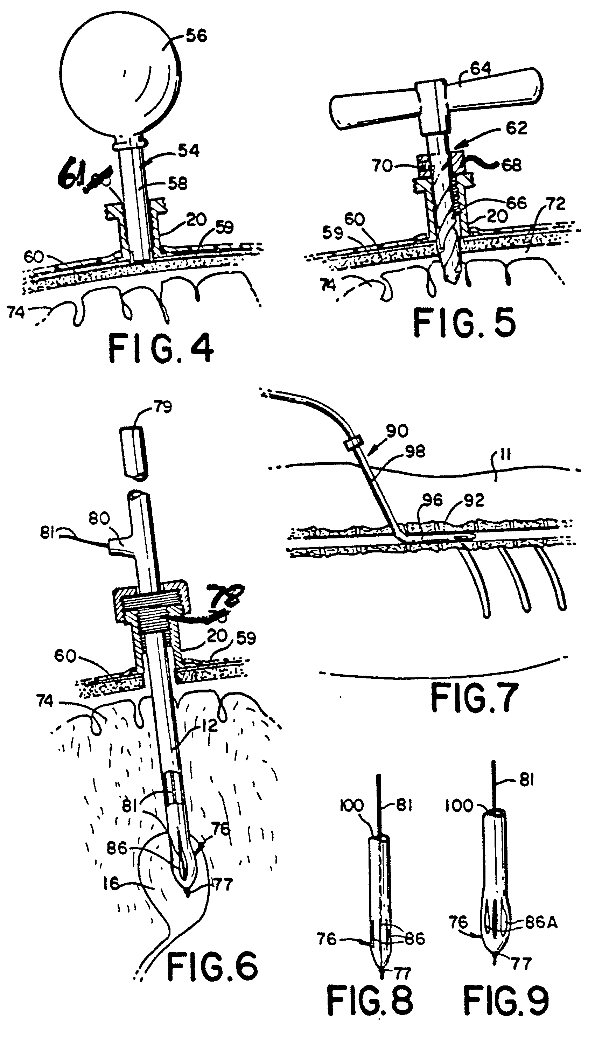

Once the harness 24 and, therefore, the guide means 20 and 22 are appropriately positioned with respect to the head of patient 11, treatment is begun immediately. Referring now to Figs. 4-6, the method of utilizing the cerebral catheterization apparatus 10 of the present invention in the course of such treatment will be described in further detail. After the positioning harness 24 described in connection with Figs. 1-3 is located with respect to the head of the patient and the guide means 20 and 22 are positioned in the appropriate location with respect to the lateral ventricles 16 and 18 as described above, apertures are created through the skull at the position of the guide means 20 and 22. These apertures are formed in the skull 60 by means of an obturator 54 as shown in Fig. 4. The obturator 54 includes a handle portion 56 and an elongated stem portion 58 having an outside diameter fitting within the threaded internal bore 61 of the respective guide means 20 or 22. The obturator 54 is utilized to pierce the scalp 59 of the patient and to remove a core of that scalp so as to expose the underlying skull 60. After the core has been removed and the skull 60 has been exposed, the obturator 54 is removed from the guide means 20 or 22 as the case may be and the next step of the present method may begin.

This next step is shown in Fig. 5 in which a drill means 62 is shown. The drill means 62 includes a handle portion 64 and a downwardly extending drill bit 66. In accordance with the preferred embodiment of the present invention, the drill means further includes a stop 68 concentrically located about the outer periphery of the drill bit 66 and fixed with respect to the drill bit 66 by means of a set screw 70. Operation of the handle portion 64 of the drill means 62 causes an aperture to be formed in the skull 60 as well as in the underlying pia layer 72. The stop means 68 is provided to prevent the insertion of the drill bit 66 into an unnecessarily large portion of soft brain tissue 74. When the drill bit 66 is inserted to the appropriate depth, the stop means 68 abuts the upper surface of the guide means 20 or 22 as shown in Fig. 5 to control the insertion depth.

After the scalp 58 has been pierced and an aperture has been formed in the skull 60, the drill means 62 is removed from the guide means 20 and the third and next step of the present method may begin.

In this next step, a catheter means such as shown at 12 in Fig. 6, is inserted into the threaded internal bore 61 of the guide means 20 or 22. The catheter means 12 includes a tip portion 76 which is inserted into the lateral ventricle such as shown at 16 in Fig. 6. With the insertion of the catheter tip 76 into the lateral ventricle, treatment in accordance with the aforementioned patents and patent applications may begin and nutrient emulsion may be circulated through the ventricle.

The catheter means 12 includes a threaded external portion 78 which mates with and which is threadedly engaged with respect to the threaded internal bore 61 of the guide means 20 or 22 and external indicia which indicate the depth of insertion. The rotational position of the catheter means 12 with respect to the guide means 20 thus controls and provides a visual indication of the insertion depth of the catheter means 12. The proper insertion depth is determined by reference to the aforementioned indicia on the tongues 40a and 40b of the vertical strap 40. These indicia are scales to correspond to indicia on the catheter means so that the catheter insertion distance can be read from tongues 40a and/or 40b. This is possible due to the precise placement of the horizontal and vertical straps in the harness, and their known relative positions with respect to the lateral ventricles. It is therefore unnecessary for the user to have any particular knowledge of brain anatomy in order to properly insert the catheter once the harness is properly positioned. When the catheter means 12 is at the proper insertion depth, thumb screws 14 and 15 (Fig. 1) may be tightened so as to fix the catheter means 12 at the appropriate depth.

The catheter means 12 and 14 each include a first port 79 through which nutrient emulsion is caused to flow and a second or pressure measurement port 80 through which intracranial pressure is monitored. Also passing through the pressure measurement port 80 is a wire means 81 which is coupled to the extreme end 77 of the catheter tip 76 for reasons which will become apparent below. The catheter means 12 is preferably made of semi-malleable plastic and is provided with multiple slit ports 86 at the tip 76 thereof. The slit ports 86 are spaced about the peripheral wall of the tip 76 of the catheter means 12.

To complete the treatment with the apparatus of the present invention, it is necessary that the nutrient emulsion which is inserted into the lateral brain ventricles be removed from the spinal subarachnoid space or, alternatively, from the cisterna magna. In order to facilitate removal of the nutrient emulsion from the subarachnoid space an improved lumbar catheter is described in connection with Fig. 7. As shown in Fig. 7, a lumbar catheter shown generally at 90 is inserted into the spinal column 92 of the patient 11. The lumbar catheter 90 includes a tip portion 96 and a body portion 98. The lumbar catheter 90 is made of semi-malleable plastic and is flexible at least at its inserted tip portion 96 so as to bend and protrude from the subarachnoid space of the spinal column 92 and project through the body wall. The lumbar catheter 90 is inserted into the subarachnoid space by first inserting a knife cannula into a hollow tube and using it to penetrate the skin, muscle and dura. When the tip of the knife and hollow tube are inserted into the subarachnoid space, the knife is withdrawn and the catheter is inserted into the hollow tube.

As shown in Figs. 8 and 9 and in accordance with an aspect of the present invention, both the cerebral catheters 12 and 14 and the lumbar catheter 90 include a thin wire 81 traversing through the central bore 100 thereof. The thin wire 84 is fixed to the extreme end portion 77 of the catheter means. As mentioned above and as shown in Figs. 8 and 9, peripheral slit ports 86 are provided around the peripheral surface of the catheter tip 76. As shown in Fig. 8, the catheter tip 76 is initially in an undeformed position and the peripheral slits 86 are closed, thus making insertion of the catheter means relatively simple. After the catheter tip 76 has been inserted, the central wire 81 is moved to a retracted position, thus deforming the catheter tip 76 and expending the radial dimension of the catheter tip 76 as shown in Fig. 9. Such a result has the effect of expanding the slits 86 and transforming them to the more enlarged apertures 86A shown in Fig. 9. Such a result improves the flow of fluid emulsion both into and from the catheter means 12 or 90 as the case may be.

## Claims

1. A cerebral catheterization apparatus for rapidly inserting a cerebral perfusion catheter means (12, 13) into the lateral brain ventricle of a patient to permit rapid delivery of oxygenated nutrient to the cerebro-spinal fluid of the patient including an appliance (10) for engaging the head, and guide means (20, 22) coupled to said appliance for receiving and directing said catheter means to the lateral brain ventricle, characterized in that the guide means (20, 22) is mounted on a harness (24) positionable with respect to the head of the patient for positioning said guide means with respect to the head such that said catheter means (12, 13) may be reliably inserted into said lateral ventricle.

2. A catheterization apparatus according to claim 1, characterized in that said catheter means includes a tube having a tip portion (76) having a plurality of outlet openings (86) therein for perfusing said oxygenated nutrient.

3. A catheterization apparatus according to claim 2, characterized in that said tube further includes a threaded portion (78) about at least a portion of the periphery thereof and in that the guide means (20, 22) has a mounting having a threaded internal bore (61) mating with said threaded portion (78) of said tube, the rotational position of said catheter means with respect to said guide means controlling the insertion depth of said catheter means into said ventricle.

4. A catheterization apparatus according to claim 3, characterized in that a locking means (14, 15) is coupled to the mounting for selectively fixing the position of said catheter means with respect to said guide means.

5. A catheterization apparatus according to claims 2 to 4, characterized in that tip portion (76) is formed of semi-malleable plastic material, in that the outlet openings are a plurality of normally closed outlet slits situated about the periphery of said tip portion, and in that a wire means (81) is coupled to said tip portion and traverses an internal passageway within the tube for deforming said tip portion and for opening said outlet slits to form outlet openings through which said oxygenated nutrient flows when said wire means is in a retracted position.

6. A catheterization apparatus according to any preceding claim, characterized in that the catheter means includes catheter depth insertion indicia for indicating the depth of insertion of said catheter.

7. A catheterization apparatus according to any preceding claim, characterized in that the harness includes a horizontal strap (26) for encircling the head of the patient, the vertex of the skull being above said horizontal strap, a vertical transverse strap (40) coupled to said horizontal strap, said vertical transverse strap generally lying above the coronal suture of said patient, said guide means being mounted thereon.

8. A catheterization apparatus according to claim 7, characterized in that a vertical medial strap (44) is coupled to said horizontal strap (26) in an anterior location and in a posterior location and is further coupled to said vertical transverse strap (40) for restraining said vertical transverse strap in an appropriate anterior-to-posterior position above the coronal suture.

9. A catheterization apparatus according to claim 8, characterized in that the vertical medial strap is coupled to said vertical transverse strap at a point generally above the bregma of the patient.

10. A catheterization apparatus according to any of claims 7 to 9, characterized in that the vertical transverse strap includes adjustment indicia to indicate its relative position to said horizontal strap.

11. A catheterization apparatus according to claim 10, characterized in that the indicia indicate the depth of required insertion of said catheter means for location of its tip in a lateral ventricle.

## Revendications

1. Appareillage de cathétérisme cérébral, destiné à introduire rapidement un cathéter de perfusion cérébrale (12, 13) dans le ventricule latéral du cerveau d'un patient pour permettre de délivrer rapidement une substance nutritive oxygénée au liquide cérébro-spinal du patient, comprenant un dispositif (10) qui se fixe à la tête, et des moyens de guidage (20, 22) couplés à ce dispositif et destinés à recevoir et à diriger lesdits cathéters vers le ventricule latéral du cerveau, caractérisé en ce que les moyens de guidage (20, 22) sont montés sur un harnais (24) qui peut être positionné par rapport à la tête du patient pour positionner lesdits moyens de guidage par rapport à la tête de telle manière que lesdits cathéters (12, 13) puissent être introduits fiablement dans ledit ventricule latéral.

2. Appareillage de cathétérisme selon la revendication 1, caractérisé en ce que lesdits cathéters comprennent un tube ayant une partie de pointe (76) munie d'une pluralité d'ouvertures de sortie (86) servant à perfuser ladite substance nutrivite oxygénée.

3. Appareillage de cathétérisme selon la revendication 2, caractérisé en ce que ledit tube comprend en outre une portion filetée (78) qui entoure au moins une portion de sa périphérie et en ce que les moyens de guidage (20, 22) portent une monture possédant une lumière intérieure filetée (61) qui s'accouple à ladite portion filetée (78) dudit tube, la position angulaire desdits cathéters par rapport auxdits moyens de guidage commandant la profondeur d'enfoncement desdits cethéters dans ledit ventricule.

4. Appareillage de cathétérisme selon la revendication 3, caractérisé en ce que des moyens de blocage (14, 15) sont couplés à la monture pour fixer sélectivement la position desdits cathéters par rapport auxdits moyens de guidage.

5. Appareillage de cathétérisme selon les revendication 2 à 4, caractérisé en ce que la partie de pointe (76) est faite d'une matière plastique semi-malléable, en ce que les ouvertures de sortie sont constituées par une pluralité de fentes de sortie normalement fermées, situées sur le tour de la périphérie de ladite portion de pointe, et en ce qu'un fil (81) est couplé à ladite portion de pointe et passe dans un passage interne formé à l'intérieur du tube pour déformer ladite portion de pointe et pour ouvrir lesdites fentes de sortie afin de former des ouvertures de sortie à travers lesquelles ladite substance nutrivite oxygénée s'écoule lorsque ledit fil est dans une position rétractée.

6. Appareillage de cathétérisme selon une quelconque des revendications précédentes, caractérisé en ce que le cathéter comprend des repères de profondeur d'enfoncement du cathéter pour indiquer la profondeur d'enfoncement dudit cathéter.

7. Appareillage de cathétérisme selon une quelconque des revendications précédentes, caractérisé en ce que le harnais comprend une sangle horizontale (26) servant à encercler la tête du patient, le sommet du crâne se trouvant au-dessus du niveau de ladite sangle latérale, une sangle verticale transversale (40) accouplée à ladite sangle horizontale, ladite sangle transversale verticale se trouvant sensiblement au-dessus de la suture coronale dudit patient, lesdits moyens de guidage étant montée sur cette sangle.

8. Appareillage de cathétérisme selon la revendication 7, caractérisé en ce qu'une sangle médiane verticale (44) est couplée à ladite sangle horizontale (26) dans une position antérieure et une position postérieure et est en outre couplée à ladite sangle transversale verticale (40) pour retenir ladite sangle transversale verticale dans une position antéro-postérieure appropriée au-dessus de la suture coronale.

9. Appareillage de cathétérisme selon la revendication 8, caractérisé an ce que la sangle médiane verticale est couplée à ladite sangle transversale verticale en un point situé à peu près au-dessus du bregma du patient.

10. Appareillage de cathétérisme selon une quelconque des revendications 7 à 9, caractérisé en ce que la sangle transversale verticale comprend des repères de réglage pour indiquer sa position relative par rapport à ladite sangle latérale.

11. Appareillage de cathétérisme selon la revendication 10, caractérisé an ce que les repères indiquent la profondeur à laquelle ledit cathéter doit être enfoncée pour placer sa pointe dans un ventricule latéral.

**Ansprüche**

1. Cerebralkatheterisiervorrichtung zum raschen Einführen einer cerebralen Perfusionskathetereinrichtung (12, 13) in den lateralen Gehirnventrikel eines Patienten, um eine rasche Zuführung von mit Sauerstoff angereicherten Nährstoffen zur cerebro-spinalen Flüssigkeit des Patienten zu erlauben, mit einer Vorrichtung (10) zum Anlegen am Kopf und einer mit ihr gekuppelten Führungseinrichtung (20, 22) zum Aufnehmen und Lenken der Kathetereinrichtung zum lateralen Gehirnventrikel, dadurch gekennzeichnet, daß die Führungseinrichtung (20, 22) an einem relativ zum Kopf des Patienten positionierbaren Geschirr (24) angebracht ist, um die Führungseinrichtung relativ zum Kopf in Position zu bringen, derart, daß die Kathetereinrichtung verläßlich in den lateralen Ventrikel eingeführt werden kann.

2. Katheterisiervorrichtung nach Anspruch 1, dadurch gekennzeichnet, daß die Kathetereinrichtung eine Röhre mit einem Spitzenteil (76) aufweist, der eine Mehrzahl von Auslaßöffnungen (86) darin zum Perfundieren des mit Sauerstoff angereicherten Nährstoffes hat.

3. Katheterisiervorrichtung nach Anspruch 2, dadurch gekennzeichnet, daß die Röhre weiters einen Gewindeteil (78) auf zumindest einem Abschnitt ihres Umfanges aufweist, und daß die Führungseinrichtung (20, 22) einen Aufbau mit einer Innengewinde-Bohrung (61) hat, die mit dem Gewindeteil (78) der Röhre zusammenpaßt, wobei die Drehpositionierung der Kathetereinrichtung relativ zur Führungseinrichtung die Einführtiefe der Kathetereinrichtung in den Ventrikel steuert.

4. Katheterisiervorrichtung nach Anspruch 3, dadurch gekennzeichnet, daß mit dem Aufbau eine Arretiereinrichtung (14, 15) zum selektiven Feststellen der Position der Kathetereinrichtung relativ zur Führungseinrichtung gekuppelt ist.

5. Katheterisiervorrichtung nach den Ansprüchen 2 bis 4, dadurch gekennzeichnet, daß der Spitzenteil (76) aus halbschmiegsamen Kunststoffmaterial gebildet ist, daß die Auslaßöffnungen eine Mehrzahl von normalerweise geschlossenen Auslaßschlitzen sind, die um den Umfang des Spitzenteils herum angeordnet sind, und daß eine Drahteinrichtung (81) mit dem Spitzenteil gekuppelt ist und den inneren Kanal innerhalb der Röhre durchsetzt, um den Spitzenteil zu verformen und die Auslaßschlitze zu öffnen, um die Auslaßöffnungen zu bilden, durch das der mit Sauerstoff angereicherte Nährstoff strömt, wenn sich die Drahteinrichtung in einer zurückgezogenen Stellung befindet.

6. Katheterisiervorrichtung nach einem der vorhergehenden Ansprüche, dadurch gekennzeichnet, daß die Kathetereinrichtung Kathetereinführtiefen-Marken zur Anzeige der Tiefe der Einführung des Katheters aufweist.

7. Katheterisiervorrichtung nach einem der vorhergehenden Ansprüche, dadurch gekennzeichnet, daß der Harnisch einen horizontalen Riemen (26) zum Umschließen des Kopfes des Patienten, wobei sich der Scheitel des Schädels oberhalb des horizontalen Riemens befindet, und einen mit dem horizontalen Riemen gekuppelten vertikalen Querriemen (40) aufweist, der allgemein oberhalb der Kranznaht des Patienten liegt, wobei die Führungseinrichtung daran angebracht ist.

8. Katheterisiervorrichtung nach Anspruch 7, dadurch gekennzeichnet, daß ein vertikaler medialer Riemen (44) mit dem horizontalen Riemen (26) an einer anterioren Stelle und an einer posterioren Stelle und weiters mit dem vertikalen Querriemen (40) gekuppelt ist, um den vertikalen Querriemen in einer geeigneten Anterior-Posterior-Ausrichtung oberhalb der Kranznaht festzuhalten.

9. Katheterisiervorrichtung nach Anspruch 8, dadurch gekennzeichnet, daß der vertikale mediale Riemen mit dem vertikalen Querriemen an einer Stelle allgemein oberhalb des Bregmas des Patienten gekuppelt ist.

10. Katheterisiervorrichtung nach einem der Ansprüche 7 bis 9, dadurch gekennzeichnet, daß der vertikale Querriemen Einstellmarken zur Anzeige seiner Relativposition relativ zum horizontalen Riemen aufweist.

11. Katheterisiervorrichtung nach Anspruch 10, dadurch gekennzeichnet, daß die Marken die Tiefe der erforderlichen Einführung der Kathetereinrichtung zur Anbringung seiner Spitze in einem lateralen Ventrikel anzeigen.

FIG. 1

**FIG. 2**

**FIG. 3**

FIG. 4

FIG. 5

FIG. 6

FIG. 7

FIG. 8     FIG. 9